# EUROPEAN PATENT APPLICATION

(11) **EP 4 749 285 A1**
(43) Date of publication of application: **27.05.2026**
(21) Application number: 25216574.1
(22) Date of filing: 18.11.2025
(51) Int. Cl.: G01N 33/543, A61B 5/00

(54) **DETECTION OF BIOMARKERS IN BIOLOGICAL SAMPLES**

(30) Priority: 21.11.2024 US 202463723189 P
(71) Applicant: Outsense Diagnostics Ltd., Tel-Aviv 4791011 (IL)
(72) Inventor: KAPP-BARNEA, Yaara, 4480500 Nirit (IL); HADASSI, Guy, 6437423 Tel Aviv (IL); TREIBITZ, Tali, 70300 Beer Yaacov (IL)
(74) Representative: Boult Wade Tennant LLP

(57) **Abstract**

Apparatus and methods are provided for use with a biological sample from a subject. A substrate (26) includes a plurality of biosynthetic molecules (44) disposed on the substrate, the biosynthetic molecules (44) configured to undergo a biochemical interaction in the presence of a given biomarker within the biological sample. A converter (46) is configured to generate electrical energy in response to the biochemical interaction occurring, and a wireless transmitter (52) is configured to transmit a signal that is indicative of the electrical energy that is generated. Other applications are also described.

## Description

### CROSS-REFERENCE TO RELATED APPLICATIONS

The present application claims priority from U.S. Provisional Patent Application No. 63/723,189 to Kapp-Barnea et al., filed November 21, 2024, entitled "Detection of Biomarkers in Bodily Emissions," which is incorporated herein by reference.

### FIELD OF EMBODIMENTS

Some applications generally relate to analysis of biological samples, e.g., bodily emissions. Specifically, some applications relate to apparatus and methods for detecting biomarkers in a biological sample, e.g., a bodily emission.

### BACKGROUND

There are substances that may be found in a human bodily emissions that are indicative of certain conditions. Some of these conditions are applicable to both male and female subjects. For example, a subject's urine or fecal matter may contain one or more physical properties, chemical compounds, and/or microscopic components indicative of an underlying condition.

### SUMMARY OF EMBODIMENTS

In some embodiments, a substrate includes one or more biosynthetic molecules, which are configured to undergo a biochemical interaction in the presence of a given biomarker. In some applications, the substrate additionally includes a converter, which is configured to convert a form of energy generated by the biochemical interaction to electrical energy. For some embodiments, the substrate additionally includes an analog-to-digital converter to convert a signal generated by the converter to a digital signal. For some applications, electrical components within the substrate are powered by a power source (e.g., a battery, a miniature battery, a capacitor, a chemical energy storage device, a nanogenerator (such as a piezoelectric, or triboelectric nanogenerator), etc.), which in some examples is degradable (e.g., biodegradable) and/or disposable. For some applications, the battery powers a miniature wireless transmitter, which in some examples is degradable (e.g., biodegradable) and/or disposable, and which is configured to transmit an electromagnetic signal, e.g., a radiowave signal, such as a UHF radiowave signal, indicating whether or not the biosynthetic molecule has undergone a biochemical interaction within the biomarker and/or indicating a concentration of the biomarker (e.g., based on the strength of the electrical current and/or voltage that is generated). For some applications, transmitter transmits the signal using a short-range wireless technology standard, such as Bluetooth^{®}. For some applications, the transmitter transmits the signal using Zigbee^{®} and/or near-field communication protocols.

For some applications, the biosynthetic molecules include one or more of an aptamer, a molecularly imprinted polymer, an antibody, a phage, and/or a different type of biosynthetic molecule. In some examples, the biochemical interaction results in electrochemical potential changes, spatial conformational changes (i.e., kinetic changes) and/or thermal changes (e.g., via an exothermic reaction or an endothermic reaction). The electrochemical potential changes, spatial conformational changes and/or thermal changes are converted to an electronic signal. For some applications, the converter is a pyroelectric converter, which is configured to convert thermal changes (e.g., heat generated or absorbed) by the biochemical interaction to electrical energy. Alternatively or additionally, the converter is a piezoelectric converter, which is configured to convert mechanical energy generated by the biochemical interaction (e.g., spatial conformational changes) to electrical energy. For some applications, the converter is a structural field-effect transistor and/or a potentiostat, which configured to convert electrochemical potential changes generated by the biochemical interaction to electrical energy.

For some applications, the substrate (including all of the components, e.g., the electronic components) on the substrate is entirely degradable (e.g., biodegradable), and/or disposable. For some applications, electronic components on the substrate (e.g., a converter, an analog-to-digital converter, a power source, and/or a transmitter) include biomaterials and/or organic materials that are degradable (e.g., biodegradable), and/or disposable.

There is therefor provided, in accordance with some embodiments of the present disclosure, the following examples, at least some of which are independent aspects of the present disclosure.

According to an independent aspect, the present disclosure includes Example 1:
Example 1. An apparatus for use with a biological sample from a subject, the apparatus comprising:
   a substrate comprising:
   a plurality of biosynthetic molecules disposed on the substrate, the biosynthetic molecules configured to undergo a biochemical interaction in the presence of a given biomarker within the biological sample;
   a converter configured to generate electrical energy in response to the biochemical interaction occurring; and
   a wireless transmitter configured to transmit a signal that is indicative of the electrical energy that is generated.
Example 2. The apparatus according to example 1, wherein the substrate comprises toilet paper that is configured to receive feces and/or urine of the subject within reactive proximity of the biosynthetic molecules in response to the subject interacting with the toilet paper.
Example 3. The apparatus according to example 1, wherein the substrate is configured to receive one or more emissions of the subject selected from the group consisting of: saliva, sweat, nasal mucus, mucus, phlegm, sweat, and tears within reactive proximity of the biosynthetic molecules in response to the subject interacting with the substrate.
Example 4. The apparatus according to example 1, wherein the substrate comprises a substrate selected from the group consisting of: toilet paper, a tampon, a sanitary product, and a flushable pad, and wherein the substrate is configured to receive vaginal fluids of the subject within reactive proximity of the biosynthetic molecules in response to the subject interacting with substrate.
Example 5. The apparatus according to example 1, wherein the substrate is configured to receive the biological sample within reactive proximity of the biosynthetic molecules in response to the subject interacting with substrate, the biological sample being selected from the group consisting of: volatile organic compounds that are present in the subject's body, epidermal tissue fluids, mucosal tissue fluids, and extracellular matrix (ECM).
Example 6. The apparatus according to example 1, wherein the wireless transmitter has a thickness of less than 500 microns.
Example 7. The apparatus according to example 1, wherein the biosynthetic molecules comprise one or more biosynthetic molecules selected from the group consisting of: an aptamer, a molecularly imprinted polymer, an antibody, and a phage.
Example 8. The apparatus according to example 1, wherein the substrate further comprises an analog-to-digital converter configured to convert the electrical energy generated by the converter to a digital signal.
Example 9. The apparatus according to any one of examples 1-8, wherein the substrate further comprises a power source.
Example 10. The apparatus according to example 9, wherein the power source comprises a battery having a thickness of less than 500 microns.
Example 11. The apparatus according to any one of examples 1-8, wherein the apparatus is for use with a toilet bowl, wherein the substrate is configured to be placed in the toilet bowl.
Example 12. The apparatus according to example 11, wherein the apparatus is for use with a sensor module disposed within the toilet bowl and wherein the wireless transmitter configured to transmit the signal by transmitting a signal that is detectable by the sensor module.
Example 13. The apparatus according to example 11, wherein the biosynthetic molecules are configured to undergo the biochemical interaction in the presence of the given biomarker within the biological sample within water within the toilet bowl.
Example 14. The apparatus according to any one of examples 1-8, wherein the biosynthetic molecules are configured to undergo a biochemical interaction that generates thermal changes in the presence of the given biomarker.
Example 15. The apparatus according to example 14, wherein the converter comprises a pyroelectric converter that is configured to convert the thermal changes generated by the biochemical interaction to electrical energy.
Example 16. The apparatus according to any one of examples 1-8, wherein the biosynthetic molecules are configured to undergo a biochemical interaction that generates spatial conformational changes in the presence of the given biomarker.
Example 17. The apparatus according to example 16, wherein the converter comprises a piezoelectric converter that is configured to convert the spatial conformational changes generated by the biochemical interaction to electrical energy.
Example 18. The apparatus according to any one of examples 1-8, wherein the biosynthetic molecules are configured to undergo a biochemical interaction that generates electrochemical potential changes in the presence of the given biomarker.
Example 19. The apparatus according to example 18, wherein the converter comprises a structural field-effect transistor configured to convert the electrochemical potential changes generated by the biochemical interaction to electrical energy.
Example 20. The apparatus according to example 18, wherein the converter comprises a potentiostat configured to convert the electrochemical potential changes generated by the biochemical interaction to electrical energy.
Example 21. The apparatus according to any one of examples 1-20, wherein the biosynthetic molecules are configured to undergo a biochemical interaction in the presence of iodine within the biological sample.
Example 22. The apparatus according to any one of examples 1-20, wherein the biosynthetic molecules are configured to undergo a biochemical interaction in the presence of a given pathogen within the biological sample.
Example 23. The apparatus according to any one of examples 1-20, wherein the biosynthetic molecules are configured to undergo a biochemical interaction in the presence of one or more inflammatory biomarkers within the biological sample.
Example 24. The apparatus according to any one of examples 1-20, wherein the biosynthetic molecules are configured to undergo a biochemical interaction in the presence of human chorionic gonadotropin (hCG).
Example 25. The apparatus according to any one of examples 1-20, wherein the biosynthetic molecules are configured to undergo a biochemical interaction in the presence of a biomarker within the biological sample that is indicative of whether a female subject is pre- or post-menopausal, the biomarker being selected from the group consisting of: 6-sulfatoxymelatonin (aMT6s), Follicle-stimulating hormone (FSH), Estrone (E1), and Estradiol (E2).
Example 26. The apparatus according to any one of examples 1-20, wherein the biosynthetic molecules are configured to undergo a biochemical interaction in the presence of a biomarker within the biological sample that is indicative of a female subject's menstrual cycle, the biomarker being selected from the group consisting of: pregnanediol glucuronide (PdG), and luteinizing hormone (LH).
Example 27. The apparatus according to any one of examples 1-20, wherein the biosynthetic molecules are configured to undergo a biochemical interaction in the presence of urinary prostate-specific antigen (PSA) within the biological sample.
Example 28. The apparatus according to any one of examples 1-20, wherein the biosynthetic molecules are configured to undergo a biochemical interaction in the presence of one or more biomarkers within the biological sample that are indicative of a likelihood of a male subject suffering from prostate cancer and/or from benign prostatic hyperplasia (BPH), the one or more biomarkers being selected from the group consisting of: urinary polyamines, and urinary volatile organic compounds.
Example 29. The apparatus according to any one of examples 1-20, wherein the biosynthetic molecules are configured to undergo a biochemical interaction in the presence of one or more biomarkers within the biological sample that are indicative of a likelihood of a subject suffering from cancer, autoimmune disease, and/or inflammation, the one or more biomarkers being selected from the group consisting of: urinary polyamines, and urinary volatile organic compounds.
Example 30. The apparatus according to any one of examples 1-20, wherein the biosynthetic molecules are configured to undergo a biochemical interaction in the presence of one or more biomarkers within the biological sample that are indicative of a likelihood of a subject suffering from a sexually-transmitted disease, the one or more biomarkers being selected from the group consisting of: urinary polyamines, and urinary volatile organic compounds.
Example 31. The apparatus according to any one of examples 1-20, wherein the biosynthetic molecules are configured to undergo a biochemical interaction in the presence of B-Type Natriuretic Peptide within urine of the subject.
Example 32. The apparatus according to any one of examples 1-20, wherein the biosynthetic molecules are configured to undergo a biochemical interaction in the presence of troponin within urine of the subject.

According to an independent aspect, the present disclosure includes Example 33:
Example 33. A method comprising:
   placing a biological sample from a subject in reactive proximity with a plurality of biosynthetic molecules disposed on a substrate, the biosynthetic molecules configured to undergo a biochemical interaction in the presence of a given biomarker within the biological sample;
   causing the biomarker, if present within the biological sample, to undergo the biochemical interaction with the biosynthetic molecules such as to generate electrical energy; and
   causing a transmitter disposed upon the substrate to generate a signal indicative of the generated electrical energy.
Example 34. The method according to example 33, wherein the substrate includes toilet paper and wherein placing the biological sample from the subject in reactive proximity with the biosynthetic molecules comprises bringing feces and/or urine of the subject into reactive proximity with the biosynthetic molecules by the subject interacting with the toilet paper.
Example 35. The method according to example 33, wherein placing the biological sample from the subject in reactive proximity with the biosynthetic molecules comprises bringing one or more emissions of the subject selected from the group consisting of: saliva, sweat, nasal mucus, mucus, phlegm, sweat, and tears into reactive proximity with the biosynthetic molecules by the subject interacting with the substrate.
Example 36. The method according to example 33, wherein the substrate includes a substrate selected from the group consisting of: toilet paper, a tampon, a sanitary product, and a flushable pad, and wherein placing the biological sample from the subject in reactive proximity with the biosynthetic molecules comprises bringing vaginal fluids of the subject into reactive proximity with the biosynthetic molecules by the subject interacting with the substrate.
Example 37. The method according to example 33, wherein placing the biological sample from the subject in reactive proximity with the biosynthetic molecules comprises bringing the biological sample within reactive proximity of the biosynthetic molecules by the subject interacting with the substrate, the biological sample being selected from the group consisting of: volatile organic compounds that are present in the subject's body, epidermal tissue fluids, mucosal tissue fluids, and extracellular matrix (ECM).
Example 36. The method according to example 33, wherein the biosynthetic molecules include one or more biosynthetic molecules selected from the group consisting of: an aptamer, a molecularly imprinted polymer, an antibody, and a phage.
Example 38. The method according to example 33, wherein causing the biomarker, if present within the biological sample, to undergo the biochemical interaction with the biosynthetic molecules comprises placing the substrate in a toilet bowl.
Example 39. The method according to any one of examples 33-38, wherein causing the biomarker, if present within the biological sample, to undergo the biochemical interaction with the biosynthetic molecules comprises causing the biosynthetic molecules to undergo a biochemical interaction that generates thermal changes in the presence of the given biomarker.
Example 40. The method according to example 39, wherein causing the biomarker, if present within the biological sample, to undergo the biochemical interaction with the biosynthetic molecules such as to generate electrical energy the converter comprises converting the thermal changes generated by the biochemical interaction to electrical energy using a pyroelectric converter.
Example 41. The method according to any one of examples 33-38, wherein causing the biomarker, if present within the biological sample, to undergo the biochemical interaction with the biosynthetic molecules comprises causing the biosynthetic molecules to undergo a biochemical interaction that generates spatial conformational changes in the presence of the given biomarker.
Example 42. The method according to example 41, wherein causing the biomarker, if present within the biological sample, to undergo the biochemical interaction with the biosynthetic molecules such as to generate electrical energy the converter comprises converting the spatial conformational changes generated by the biochemical interaction to electrical energy using a piezoelectric converter.
Example 43. The method according to any one of examples 33-38, wherein causing the biomarker, if present within the biological sample, to undergo the biochemical interaction with the biosynthetic molecules comprises causing the biosynthetic molecules to undergo electrochemical potential changes in the presence of the given biomarker.
Example 44. The method according to example 43, wherein causing the biomarker, if present within the biological sample, to undergo the biochemical interaction with the biosynthetic molecules such as to generate electrical energy the converter comprises converting the electrochemical potential changes generated by the biochemical interaction to electrical energy using a structural field-effect transistor.
Example 45. The method according to example 43, wherein causing the biomarker, if present within the biological sample, to undergo the biochemical interaction with the biosynthetic molecules such as to generate electrical energy the converter comprises converting the electrochemical potential changes generated by the biochemical interaction to electrical energy using a potentiostat.
Example 46. The method according to any one of examples 33-45, wherein the biosynthetic molecules are configured to undergo a biochemical interaction in the presence of a given pathogen within the biological sample.
Example 47. The method according to any one of examples 33-45, wherein the biosynthetic molecules are configured to undergo a biochemical interaction in the presence of one or more inflammatory biomarkers within the biological sample.
Example 48. The method according to any one of examples 33-45, wherein the biosynthetic molecules are configured to undergo a biochemical interaction in the presence of human chorionic gonadotropin (hCG).
Example 49. The method according to any one of examples 33-45, wherein the biosynthetic molecules are configured to undergo a biochemical interaction in the presence of a biomarker within the biological sample that is indicative of whether a female subject is pre- or post-menopausal, the biomarker being selected from the group consisting of: 6-sulfatoxymelatonin (aMT6s), Follicle-stimulating hormone (FSH), Estrone (E1), and Estradiol (E2).
Example 50. The method according to any one of examples 33-45, wherein the biosynthetic molecules are configured to undergo a biochemical interaction in the presence of a biomarker within the biological sample that is indicative of a female subject's menstrual cycle, the biomarker being selected from the group consisting of: pregnanediol glucuronide (PdG), and luteinizing hormone (LH).
Example 51. The method according to any one of examples 33-45, wherein the biosynthetic molecules are configured to undergo a biochemical interaction in the presence of urinary prostate-specific antigen (PSA) within the biological sample.
Example 52. The method according to any one of examples 33-45, wherein the biosynthetic molecules are configured to undergo a biochemical interaction in the presence of one or more biomarkers within the biological sample that are indicative of a likelihood of a male subject suffering from prostate cancer and/or from benign prostatic hyperplasia (BPH), the one or more biomarkers being selected from the group consisting of: urinary polyamines, and urinary volatile organic compounds.
Example 53. The method according to any one of examples 33-45, wherein the biosynthetic molecules are configured to undergo a biochemical interaction in the presence of one or more biomarkers within the biological sample that are indicative of a likelihood of a subject suffering from cancer, the one or more biomarkers being selected from the group consisting of: urinary polyamines, and urinary volatile organic compounds.
Example 54. The method according to any one of examples 33-45, wherein the biosynthetic molecules are configured to undergo a biochemical interaction in the presence of one or more biomarkers within the biological sample that are indicative of a likelihood of a subject suffering from a sexually-transmitted disease, the one or more biomarkers being selected from the group consisting of: urinary polyamines, and urinary volatile organic compounds.
Example 55. The method according to any one of examples 33-45, wherein the biosynthetic molecules are configured to undergo a biochemical interaction in the presence of B-Type Natriuretic Peptide within urine of the subject.
Example 56. The method according to any one of examples 33-45, wherein the biosynthetic molecules are configured to undergo a biochemical interaction in the presence of troponin within urine of the subject.

According to an independent aspect, the present disclosure includes Example 57:
Example 57. An apparatus for use with a biological sample from a subject and a toilet bowl, the apparatus comprising:
   a substrate configured to be placed in the toilet bowl and come into contact with the biological sample, the substrate being configured to transmit an electromagnetic signal that is indicative of a given biomarker being present within the biological sample; and
   a sensor configured to detect the signal generated by the substrate; and
   a computer processor configured to generate an output in response to the detected signal.
Example 58. The apparatus according to example 57, wherein the substrate is configured to transmit an electromagnetic signal that is indicative of a presence of B-Type Natriuretic Peptide within urine of the subject, and wherein the computer processor is configured to generate an output indicating a likelihood of the subject suffering from congestive heart failure in response thereto.
Example 59. The apparatus according to example 57, wherein the substrate is configured to transmit an electromagnetic signal that is indicative of a presence of troponin within urine of the subject, and wherein the computer processor is configured to generate an output indicating a likelihood of the subject suffering from cardiac ischemia in response thereto.

According to an independent aspect, the present disclosure includes Example 60:
Example 60. An apparatus for use with a biological sample from a subject, the apparatus comprising:
a plurality of biosynthetic molecules, the biosynthetic molecules configured to undergo a biochemical interaction in the presence of a given biomarker within the biological sample;
a converter configured to generate electrical energy in response to the biochemical interaction occurring; and
a wireless transmitter configured to transmit a signal that is indicative of the electrical energy that is generated.

According to an independent aspect, the present disclosure includes Example 61:
Example 61. A method comprising:
placing a biological sample from a subject in reactive proximity with a plurality of biosynthetic molecules, the biosynthetic molecules configured to undergo a biochemical interaction in the presence of a given biomarker within the biological sample;
causing the biomarker, if present within the biological sample, to undergo the biochemical interaction with the biosynthetic molecules such as to generate electrical energy; and
causing a transmitter to generate a signal indicative of the generated electrical energy.

The present disclosure will be more fully understood from the following detailed description of embodiments thereof, taken together with the drawings, in which:

### BRIEF DESCRIPTION OF THE DRAWINGS

Fig. 1A is a schematic illustration of a substrate that is configured to generate an electronic signal that is indicative of the presence of biomarkers, in accordance with some applications of the present disclosure;
Fig. 1B is a schematic illustration of the substrate within a toilet bowl, in accordance with some applications of the present disclosure; and
Fig. 2 is a flowchart showing steps of analysis that is performed on a subject's biological sample, in accordance with some applications of the present disclosure.

### DETAILED DESCRIPTION OF EMBODIMENTS

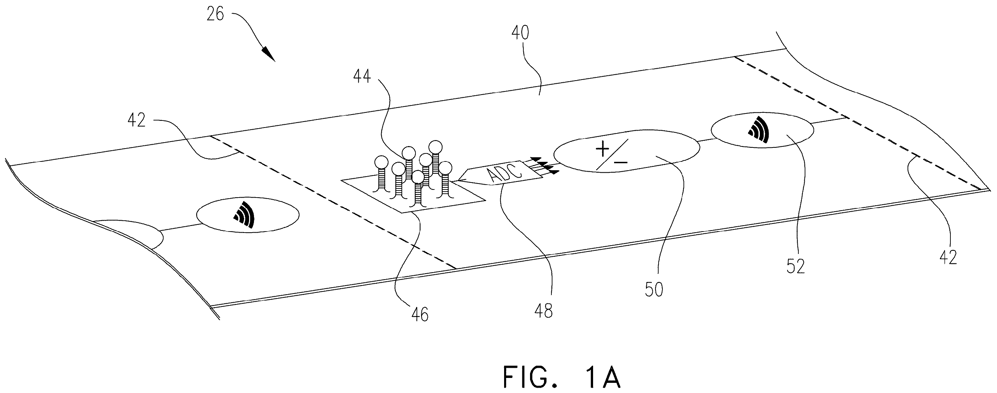

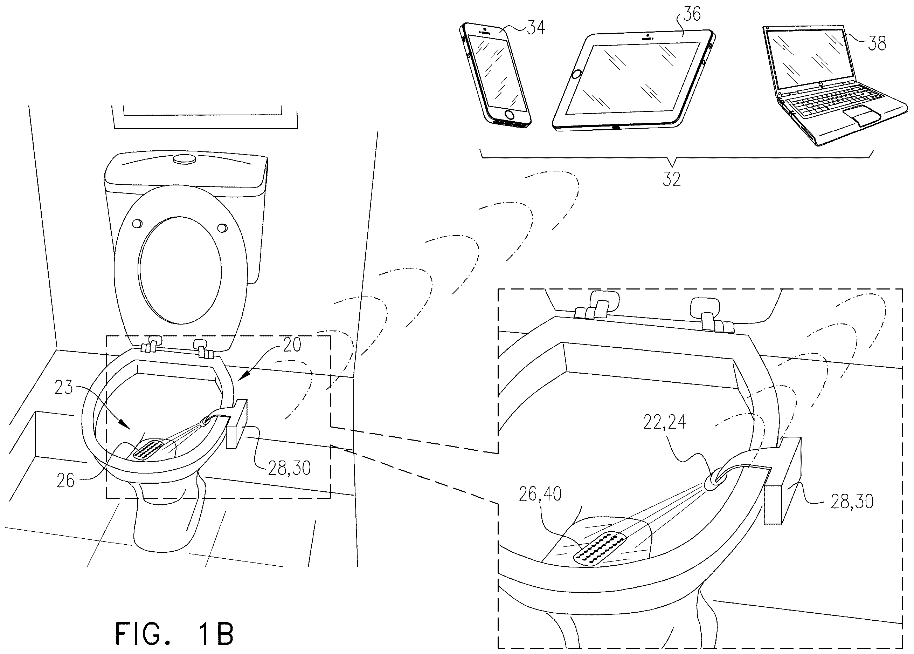

Reference is now made to Fig. 1A, which is a schematic illustration of a substrate 26 that is configured to generate an electronic signal that is indicative of the presence of biomarkers, in accordance with some applications of the present disclosure. Reference is also made to Fig. 1B, which is a schematic illustration of apparatus 20 for use with substrate 26.

For some applications, substrate 26 is flushable in a toilet bowl 23 (shown in Fig. 1B). For example, the substrate may include toilet paper 40 (as shown in Fig. 1A), wipes (such as facial wipes), tampons, flushable pads, sanitary products, and/or a different type of substrate. For some applications, a sensor module 22 is disposed within toilet bowl 23 and is configured to detect signals generated by the substrate. Alternatively or additionally, the signal generated by the substrate is communicated directly to a user interface device 32, which may include, but is not limited to, a phone 34, a tablet computer 36, a laptop computer 38, or a different sort of personal computing device.

Fig. 1A shows one full piece of toilet paper 40 within a roll of toilet paper, with perforations 42 at each end of the piece of the toilet paper. In some embodiments, the substrate includes one or more biosynthetic molecules 44, which are configured to undergo a biochemical interaction in the presence of a given biomarker. In some applications, the substrate additionally includes a converter 46, which is configured to convert a form of energy generated by the biochemical interaction to electrical energy. For some embodiments, the substrate additionally includes an analog-to-digital converter 48 to convert a signal generated by converter 46 to a digital signal. For some applications, electrical components within the substrate are powered by a miniature power source 50 (e.g., a battery, a miniature battery, a capacitor, a chemical energy storage device, a nanogenerator (such as a piezoelectric, or triboelectric nanogenerator), etc.), which in some examples is degradable (e.g., biodegradable) and/or disposable. For some applications, the power source powers a miniature wireless transmitter 52, which in some examples is degradable (e.g., biodegradable) and/or disposable, and which is configured to transmit an electromagnetic signal, e.g., a radiowave signal, such as a UHF radiowave signal, indicating whether or not the biosynthetic molecule has undergone a biochemical interaction within the biomarker and/or indicating a concentration of the biomarker (e.g., based on the strength of the electrical current and/or voltage that is generated). For some applications, transmitter transmits the signal using a short-range wireless technology standard, such as Bluetooth^{®}. For some applications, the transmitter transmits the signal using Zigbee^{®} and/or near-field communication protocols.

For some applications, substrate 26 (including all of the components, e.g., the electronic components) on the substrate is entirely degradable (e.g., biodegradable), and/or disposable. For some applications, electronic components on the substrate (e.g., converter 46, analog-to-digital converter 48, power source 50, and/or transmitter 52) include biomaterials and/or organic materials that are degradable (e.g., biodegradable), and/or disposable.

As described hereinabove, for some applications, sensor module 22, which is disposed within toilet bowl 23 is configured to detect the signal generated by the substrate. Alternatively or additionally, the signal generated by the substrate is communicated directly to a sensor within a user interface device 32, which may include, but is not limited to, a phone 34, a tablet computer 36, a laptop computer 38, or a different sort of personal computing device.

For some applications, sensor module 22 and/or the user interface device 32 communicates with a remote server, e.g., to thereby communicate with a third-party device. For example, the apparatus may communicate with a physician or an insurance company over a communication network without intervention from the subject. The physician or the insurance company may evaluate the results on the third-party device and determine whether further testing or intervention is appropriate for the subject.

For some applications, data relating to the received signals are stored in a memory. For some applications, the sensor module includes a computer processor 28 and associated memory disposed within a housing 30. For some applications, memory associated within computer processor 28 stores data relating to the received signals. Periodically, the subject may submit the stored data to a remote device at a facility, such as a healthcare facility (e.g., a physician's office, or a pharmacy) or an insurance company, and a computer processor of the remote device at the facility may then perform the above-described analysis on a batch of data that were acquired over a period of time.

For some applications, biosynthetic molecules 44 include one or more of an aptamer, a molecularly imprinted polymer, an antibody, a phage, and/or a different type of biosynthetic molecule. In some examples, the biochemical interaction results in electrochemical potential changes, spatial conformational changes (i.e., kinetic changes) and/or thermal changes (e.g., via an exothermic reaction or an endothermic reaction). The electrochemical potential changes, spatial conformational changes and/or thermal changes are converted to an electronic signal.

For some applications, converter 46 is a pyroelectric converter, which is configured to convert thermal changes (e.g., heat generated or absorbed) by the biochemical interaction to electrical energy. For example, the pyroelectric converter may include one or more of polyvinylidene fluoride, structures containing repeated oxygen octahedra surrounding another type of ion (i.e., a structure, like perovskite, with the general formula ABO3 (viz., CaTiO3,BaTi03 PbZr03, PbTiOs, niobates, and tantalates), cobalt phthalocyanine, lithium tantalite, and/or tourmaline.

Alternatively or additionally, converter 46 is a piezoelectric converter, which is configured to convert mechanical energy generated by the biochemical interaction (e.g., spatial conformational changes) to electrical energy. For example, the piezoelectric converter may include one or more of polyvinylidene fluoride, piezoelectric polysaccharides (including cellulose and chitin and/or chitosan), and piezoelectric nanofibers of glycine crystals embedded inside a carbon nanotube, e.g., a polycaprolactone carbon nanotube. For some applications, converter 46 is a structural field-effect transistor and/or a potentiostat, which configured to convert electrochemical potential changes generated by the biochemical interaction to electrical energy. For some applications, analog-to-digital converter 48 converts a signal generated by converter 46 to a digital signal.

In some embodiments, power source 50 is a miniature battery, having a thickness of less than 500 microns, and a diameter, a length, and/or a width, each of which is less than 15 mm. Similarly, in some embodiments, wireless transmitter 52 is a miniature wireless transmitter, having a thickness of less than 500microns, and a diameter, a length, and/or a width, each of which is less than 15 mm.

For some applications, the substrate is configured such that a biological sample, e.g., a bodily emission, comes into reactive proximity with biosynthetic molecules 44 by the subject wiping themselves (or being wiped) with the substrate. For example, when toilet paper is used as the substrate, the subject's feces and/or urine comes into reactive proximity with the biosynthetic molecules by the subject wiping themselves with the toilet paper. Alternatively, when toilet paper, facial wipes, and/or tissues are used as the substrate, the subject's saliva, sweat, nasal mucus, mucus, phlegm, sweat, tears, and/or other emissions from the subject come into reactive proximity with the biosynthetic molecules by the subject wiping themselves with the substrate. Further alternatively, when toilet paper, tampons, or flushable pads are used as the substrate, the subject's vaginal fluids come into reactive proximity with the biosynthetic molecules by the substrate being placed into contact with, or into the vicinity of, the subject's vagina. For some applications, volatile organic compounds that are present in the subject's body, epidermal tissue fluids, mucosal tissue fluids, and/or extracellular matrix (ECM) comes into reactive proximity with the biosynthetic molecules, for example by being placed into contact with the substrate. For some applications, the substrate is placed into the toilet bowl separately from the biological sample. For example, the subject may place the substrate in the toilet bowl before, during, and/or after urinating and/or defecating into the toilet bowl, such that the biological sample comes into reactive proximity with the biosynthetic molecules upon the substrate within the toilet bowl.

For some applications, the aqueous environment of the water within the toilet bowl causes the biomarker to react with biosynthetic molecules 44 (or causes a portion of the reaction to occur) such that the biosynthetic molecules undergo the biochemical interaction. Thus, although the biological sample comes into reactive proximity with the biosynthetic molecules by the subject wiping themselves with the substrate, the biochemical interaction (or a portion thereof) occurs only once the substrate is within the toilet bowl.

As described hereinabove, for some applications, biosynthetic molecules 44 include one or more of an aptamer, a molecularly imprinted polymer, an antibody, a phage, and/or a different type of biosynthetic molecule, which are configured to undergo a biochemical interaction in the presence of a given biomarker. Some examples of such biomarkers and the corresponding conditions are now provided.

For some applications, biosynthetic molecules 44 are configured to undergo a biochemical interaction in the presence of iodine and the computer processor is configured to detect a presence and/or concentration of iodine in a biological sample (e.g., urine) of the subject by detecting the signal generated by the substrate. For some such applications, the computer processor is configured to generate an output (e.g., on user interface device 32) indicating whether the subject's iodine intake is insufficient, adequate, and/or excessive. For example, in response to detecting a concentration of iodine within the subject's urine that is below a threshold (with the threshold being, for example, 150 microgram/liter or less), the computer processor generates an output indicting that the subject's iodine intake is insufficient; in response to detecting a concentration of iodine within the subject's urine that is within a given range (with the range being, for example, 150 - 499 microgram/liter or a subrange thereof), the computer processor generates an output indicting that the subject's iodine intake is adequate; and/or in response to detecting a concentration of iodine within the subject's urine that is above a threshold (with the threshold being, for example, 500 microgram/liter or more), the computer processor generates an output indicating that the subject's iodine intake is excessive.

For some applications, biosynthetic molecules 44 are configured to undergo a biochemical interaction in the presence of human chorionic gonadotropin (hCG) and the computer processor is configured to detect a concentration of human chorionic gonadotropin (hCG) in a biological sample (e.g., urine) of the subject by detecting the signal generated by the substrate. For some such applications, the computer processor is configured to generate an output (e.g., on user interface device 32) indicating a likelihood of a female subject being pregnant based upon the detected concentration of human chorionic gonadotropin (hCG). For example, in response to detecting a concentration of human chorionic gonadotropin (hCG) within the subject's urine that is below a threshold (with the threshold being, for example, 70 picomole/liter or less), the computer processor generates an output indicting that the subject's iodine intake is insufficient; in response to detecting a concentration of human chorionic gonadotropin (hCG) within the subject's urine that is within a given range (with the range being, for example, 70-174 picomole/liter or a subrange thereof), the computer processor generates an output indicting that the subject's pregnancy status is unclear; and/or in response to detecting a concentration of human chorionic gonadotropin (hCG) within the subject's urine that is above a threshold (with the threshold being, for example, 174 picomole/liter or more), the computer processor generates an output indicting that the subject is likely to be pregnant.

For some applications, biosynthetic molecules 44 are configured to undergo a biochemical interaction in the presence of 6-sulfatoxymelatonin (aMT6s), Follicle-stimulating hormone (FSH), Estrone (E1), and/or Estradiol (E2) and the computer processor is configured to detect a concentration of 6-sulfatoxymelatonin (aMT6s), Follicle-stimulating hormone (FSH), Estrone (E1), and/or Estradiol (E2) in a biological sample from the subject by detecting the signal generated by the substrate. For some applications, the computer processor is configured to determine a concentration of one or more of the aforementioned entities within a female subject's urine (e.g., first morning urine). For some applications, the computer processor is configured to determine a mean concentration of one or more of the aforementioned entities within the subject's urine over a given time period (e.g., a period of between one week and two months). For some such applications, the computer processor is configured to generate an output (e.g., on user interface device 32) indicating whether a female subject is pre- or post-menopause based upon the concentration or mean concentration of one or more of the aforementioned entities within the subject's urine. For example, in response to detecting a concentration of 6-sulfatoxymelatonin (aMT6s), Estrone (E1), and/or Estradiol (E2) within the subject's urine that is below a threshold or that the concentration of follicle-stimulating hormone (FSH) is above a threshold, the computer processor generates an output indicting that the subject is post-menopause; and in response to detecting a concentration of 6-sulfatoxymelatonin (aMT6s), Estrone (E1), and/or Estradiol (E2) within the subject's urine that is above a threshold or that the concentration of follicle-stimulating hormone (FSH) is below a threshold, the computer processor generates an output indicting that the subject is pre-menopause.

For some applications, biosynthetic molecules 44 are configured to undergo a biochemical interaction in the presence of pregnanediol glucuronide (PdG) and/or luteinizing hormone (LH) and the computer processor is configured to detect a concentration of pregnanediol glucuronide (PdG) and/or luteinizing hormone (LH) in a biological sample from the subject by detecting the signal generated by the substrate. For some applications, the computer processor is configured to determine a concentration of one or more of the aforementioned entities within a female subject's urine (e.g., first morning urine). For some such applications, the computer processor is configured to generate an output (e.g., on user interface device 32) indicating a current stage of a female subject's menstrual cycle based upon the concentration of one or more of the aforementioned entities within the subject's urine. For example, in response to detecting a concentration of pregnanediol glucuronide (PdG) within the subject's urine that is above a threshold, the computer processor generates an output indicting that the subject is at the luteal stage of their menstrual cycle; and in response to detecting a concentration of pregnanediol glucuronide (PdG) within the subject's urine that is below a threshold, the computer processor generates an output indicting that the subject at the follicular stage of their menstrual cycle. Alternatively or additionally, in response to detecting a concentration of luteinizing hormone (LH) within the subject's urine that is below a threshold, the computer processor generates an output indicting that the subject is at the luteal stage of their menstrual cycle; and in response to detecting a concentration of luteinizing hormone (LH) within the subject's urine that is above a threshold, the computer processor generates an output indicting that the subject is at the follicular stage of their menstrual cycle.

For some applications, biosynthetic molecules 44 are configured to undergo a biochemical interaction in the presence of urinary prostate-specific antigen (PSA) and the computer processor is configured to detect a concentration of urinary prostate-specific antigen (PSA) in urine of the subject by detecting the signal generated by the substrate. For some applications, the computer processor is configured to determine a concentration of urinary prostate-specific antigen (PSA) within a male subject's urine (e.g., first morning urine). For some such applications, the computer processor is configured to generate an output (e.g., on user interface device 32) indicating that the subject is likely to be suffering from prostate cancer and/or from benign prostatic hyperplasia (BPH) based upon the concentration of prostate-specific antigen (PSA) within the subject's urine. For some applications, the computer processor generates an output indicating that the subject should have their serum prostate-specific antigen (PSA) measured, so that the subject can be diagnosed based upon the ratio between their urinary prostate-specific antigen (PSA) and their serum prostate-specific antigen (PSA).

For some applications, biosynthetic molecules 44 are configured to undergo a biochemical interaction in the presence of one or more urinary polyamines (such as spermine) and the computer processor is configured to detect a concentration of the one or more urinary polyamines (such as spermine) in urine of the subject by detecting the signal generated by the substrate. For some applications, the computer processor is configured to determine a concentration of the one or more urinary polyamines (such as spermine) within a male subject's urine. For some applications, the computer processor is configured to determine a mean concentration of one or more of the aforementioned entities within the subject's urine over a given time period (e.g., a period of between 12 hours and 72 hours). For some such applications, the computer processor is configured to generate an output (e.g., on user interface device 32) indicating that the subject is likely to be suffering from prostate cancer and/or from benign prostatic hyperplasia (BPH) based upon the concentration and/or mean concentration of the one or more urinary polyamines (such as spermine) within the subject's urine. For some applications, biosynthetic molecules 44 are configured to undergo a biochemical interaction in the presence of one or more biomarkers that are indicative of a cancer (e.g., bladder cancer), and the computer processor is configured to detect a concentration of the biomarkers by detecting the signal generated by the substrate. For some such applications, the computer processor is configured to generate an output (e.g., on user interface device 32) indicating that the subject is likely to be suffering from the cancer based upon the concentration and/or mean concentration of the biomarker within a biological sample from the subject (such as urine).

For some applications, biosynthetic molecules 44 are configured to undergo a biochemical interaction in the presence of one or more biomarkers that are indicative of a sexually-transmitted disease and/or any microbial, parasitic and/or viral infections of the gastrointestinal tract and/or the urinary tract, and the computer processor is configured to detect a concentration of the biomarkers by detecting the signal generated by the substrate. For example, biosynthetic molecules 44 may include ococcal-specific monoclonal antibodies configured to undergo a biochemical interaction in the presence of neisseria gonorrhoeae. Alternatively or additionally, biosynthetic molecules 44 include a specific recombinant T. Pallidum antigen (e.g. TPHA , TpN 15, 17,47) configured to undergo a biochemical interaction in the presence of syphilis. Further alternatively or additionally, biosynthetic molecules 44 are configured to undergo a biochemical interaction in the presence of 3-hydroxy-2,4,4-trimethylpentyl 2-methylpropanoate, to detect trichomonas vaginalis. For some such applications, the computer processor is configured to generate an output (e.g., on user interface device 32) indicating that the subject is likely to be suffering from the sexually-transmitted disease, a gastrointestinal infection, and/or a urinary-tract infection, based upon the concentration and/or mean concentration of the biomarker within a biological sample from the subject (such as urine or feces).

For some applications, biosynthetic molecules 44 are configured to undergo a biochemical interaction in the presence of one or more biomarkers in urine such as protein (e.g., albumin) and/or creatinine. For some applications, biosynthetic molecules 44 are configured to undergo a biochemical interaction in the presence of one or more inflammatory biomarkers in stool (e.g., calprotectin) and/or urine (e.g., nitrites).

For some applications, biosynthetic molecules 44 are configured to undergo a biochemical interaction in the presence of a given type of pathogen within stool and/or urine, to identify a source of an infection. For example, biosynthetic molecules 44 are configured to undergo a biochemical interaction in the presence of pyuria, leukocyte esterase (LE), and/or nitrite within urine, to detect a urinary infection. Alternatively or additionally, biosynthetic molecules 44 are configured to undergo a biochemical interaction in the presence of calprotectin and/or lactoferrin within feces, to detect a gastrointestinal infection.

For some applications, biosynthetic molecules 44 are configured to undergo a biochemical interaction in the presence of B-Type Natriuretic Peptide within urine. For some such applications, in response to detecting B-Type Natriuretic Peptide within urine and/or in response to detecting that the concentration of B-Type Natriuretic Peptide within urine exceeds a threshold, the computer processor determines (and, optionally, generates an output indicating) a likelihood that the subject is suffering from congestive heart failure. For some applications, the computer processor generates an output indicating that the subject should be tested for congestive heart failure.

For some applications, biosynthetic molecules 44 are configured to undergo a biochemical interaction in the presence of troponin within urine. For some such applications, in response to detecting troponin within urine and/or in response to detecting that the concentration of troponin within urine exceeds a threshold, the computer processor determines (and, optionally, generates an output indicating) a likelihood that the subject is suffering from cardiac ischemia. For some applications, the computer processor generates an output indicating that the subject should be tested for cardiac ischemia.

As described hereinabove, for some applications, biosynthetic molecules 44 are configured to undergo a biochemical interaction in the presence of urinary volatile organic compounds, and the computer processor is configured to detect a concentration of the urinary volatile organic compounds by detecting the signal generated by the substrate. For some applications, biosynthetic molecules 44 are configured to undergo a biochemical interaction in the presence of urinary polyamines, and the computer processor is configured to detect a concentration of the polyamines by detecting the signal generated by the substrate. For some applications, the urinary polyamines and/or urinary volatile organic compounds are indicative of a male subject suffering from prostate cancer and/or from benign prostatic hyperplasia (BPH). Alternatively or additionally, the urinary polyamines and/or urinary volatile organic compounds are indicative of a likelihood of the subject suffering from cancer, autoimmune disease, and/or inflammation. Further alternatively or additionally, the urinary polyamines and/or urinary volatile organic compounds are indicative of a likelihood of the subject suffering from a sexually-transmitted disease.

For some applications, biosynthetic molecules 44 are configured to undergo a biochemical interaction in the presence of a biomarker within the subject's saliva, sweat, nasal mucus, mucus, phlegm, sweat, tears, vaginal emissions, and/or other emissions from the subject. For some applications, the substrate includes toilet paper, facial wipes, tampons, flushable pads, sanitary products, and/or tissues and one or more of the aforementioned emissions are brought into reactive proximity with the biosynthetic molecules by the subject interacting with the substrate, e.g., by wiping themselves with the substrate.

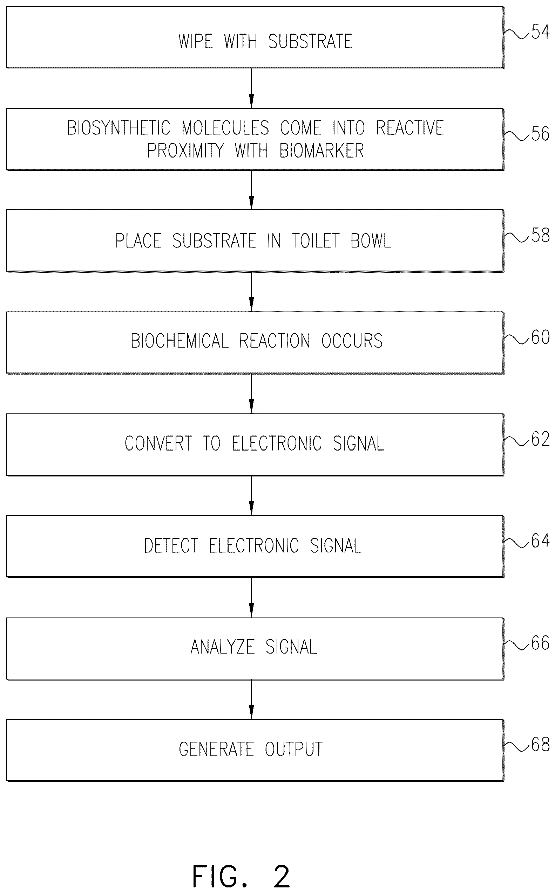

Reference is now made to Fig. 2, which is a flowchart showing steps of a method that is performed, in accordance with some applications of the present disclosure. For some applications, in a first step 54, the subject wipes a portion of their body with substrate 26, such that a biological sample is deposited onto the substrate. In some embodiments, this causes biosynthetic molecules upon the substrate to come into reactive proximity with a given biomarker within the biological sample, in step 56. For some applications, the substrate is then placed into the toilet bowl, in step 58. In some embodiments, placement of the substrate into the aqueous environment of the water within the toilet bowl causes the biomarker to undergo a biochemical interaction such as to generate an electronic signal, in step 60. It is noted that in some embodiments, the biochemical interaction occurs (such that an electronic signal is generated by the substrate) even without the substrate being placed into the aqueous environment of the water within the toilet bowl. Therefore, step 58 is shown in a dashed box to indicate that this step is optional. Further, various steps in solid boxes may be omitted and additional steps may be performed.

In step 62, the biochemical interaction is converted into an electronic signal. In some examples, the biochemical interaction results in electrochemical potential changes, spatial conformational changes (i.e., kinetic changes) and/or thermal changes (e.g., via an exothermic reaction or an endothermic reaction). The electrochemical potential changes, spatial conformational changes and/or thermal changes are converted to an electronic signal. As described hereinabove, for some applications, converter 46 is a pyroelectric converter, which is configured to convert thermal changes (e.g., heat generated or absorbed) by the biochemical interaction to electrical energy, which may be detected by a computer processor. Alternatively or additionally, converter 46 is a piezoelectric converter, which is configured to convert mechanical energy generated by the biochemical interaction to electrical energy, which may be detected by a computer processor. For some applications, analog-to-digital converter 48 converts a signal generated by converter 46 to a digital signal. For some applications, the converter 46 is a structural field-effect transistor and/or a potentiostat, which configured to convert electrochemical potential changes generated by the biochemical interaction to electrical energy, which may be detected by a computer processor.

Subsequently (in step 64), the computer processor receives the signal that is generated by the substrate. The computer processor analyzes the signal (step 66) and (in step 68) generates an output in response thereto.

As noted hereinabove, for some applications, the biological sample is deposited into the toilet bowl separately from the substrate. For example, the subject may place the substrate in the toilet bowl before, during, and/or after urinating and/or defecating into the toilet bowl, such that the biological sample comes into reactive proximity with the biosynthetic molecules upon the substrate within the toilet bowl. For applications, the subject does wipe themselves with the substrate, but the biosynthetic molecules upon the substrate are configured to undergo a biochemical interaction in the presence of a biomarker within the biological sample only once within the aqueous environment of the water within the toilet bowl. For some applications, as an alternative to or in addition to the biosynthetic molecules being disposed upon the substrate, the biosynthetic molecules are deposited into the toilet bowl via a different method. For example, cartridges containing the biosynthetic molecules may be disposed within the toilet (e.g., within the bowl itself or within the tank of the toilet) and the cartridges are configured to inject the biosynthetic molecules into the toilet bowl and/or the toilet tank.

Applications of the disclosure described herein can take the form of a computer program product accessible from a computer-usable or computer-readable medium (e.g., a non-transitory computer-readable medium) providing program code for use by or in connection with a computer or any instruction execution system, such as a computer processor of user interface device 32, computer processor 28 disposed within housing 30, or a remote cloud-based computer processor. For the purpose of this description, a computer-usable or computer readable medium can be any apparatus that can include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. For some applications, the computer-usable or computer readable medium is a non-transitory computer-usable or computer readable medium.

Examples of a computer-readable medium include a semiconductor or solid-state memory, magnetic tape, a removable computer diskette, a random-access memory (RAM), a read-only memory (ROM), a rigid magnetic disk and an optical disk. Current examples of optical disks include compact disk-read only memory (CD-ROM), compact disk-read/write (CD-R/W) and DVD. For some applications, cloud storage is used.

A data processing system suitable for storing and/or executing program code will include at least one processor (e.g., a computer processor of user interface device 32, computer processor 28 disposed within housing 30, or a remote cloud-based computer processor) coupled directly or indirectly to memory elements (e.g., a memory of user interface device 32) through a system bus. The memory elements can include local memory employed during actual execution of the program code, bulk storage, and cache memories which provide temporary storage of at least some program code in order to reduce the number of times code must be retrieved from bulk storage during execution. The system can read the inventive instructions on the program storage devices and follow these instructions to execute the methodology of the embodiments of the disclosure.

Network adapters may be coupled to the processor to enable the processor to become coupled to other processors or remote printers or storage devices through intervening private or public networks. Modems, cable modem and Ethernet cards are just a few of the currently available types of network adapters.

Computer program code for carrying out operations of the present disclosure may be written in any combination of one or more programming languages, including an object-oriented programming language such as Java, Smalltalk, C++ or the like and conventional procedural programming languages, such as the C programming language or similar programming languages.

It will be understood that the algorithms described herein can be implemented by computer program instructions. These computer program instructions may be provided to a processor of a general purpose computer, special purpose computer, or other programmable data processing apparatus to produce a machine, such that the instructions, which execute via the processor of the computer (e.g., a computer processor of user interface device 32, computer processor 28 disposed within housing 30, or a remote cloud-based computer processor) or other programmable data processing apparatus, create means for implementing the functions/acts specified in the algorithms described in the present application. These computer program instructions may also be stored in a computer-readable medium (e.g., a non-transitory computer-readable medium) that can direct a computer or other programmable data processing apparatus to function in a particular manner, such that the instructions stored in the computer-readable medium produce an article of manufacture including instruction means which implement the function/act specified in the algorithms. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide processes for implementing the functions/acts specified in the algorithms described in the present application.

In some examples, the computer processors described herein are hardware devices programmed with computer program instructions to produce a special purpose computer. For example, when programmed to perform the algorithms described herein, the computer processor acts as a special purpose bodily-emission-analysis computer processor. In some examples, the operations described herein that are performed by computer processors transform the physical state of a memory, which is a real physical article, to have a different magnetic polarity, electrical charge, or the like depending on the technology of the memory that is used.

It will be appreciated by persons skilled in the art that the present disclosure is not limited to what has been particularly shown and described hereinabove. Rather, the scope of the present disclosure includes both combinations and subcombinations of the various features described hereinabove, as well as variations and modifications thereof that are not in the prior art, which would occur to persons skilled in the art upon reading the foregoing description.

## Claims

1. An apparatus for use with a biological sample from a subject, the apparatus comprising:
a substrate (26) comprising:
a plurality of biosynthetic molecules (44) disposed on the substrate (26), the biosynthetic molecules (44) configured to undergo a biochemical interaction in the presence of a given biomarker within the biological sample;
a converter (46) configured to generate electrical energy in response to the biochemical interaction occurring; and
a wireless transmitter (48) configured to transmit a signal that is indicative of the electrical energy that is generated.

2. The apparatus according to claim 1, wherein the substrate (26) comprises toilet paper that is configured to receive feces and/or urine of the subject within reactive proximity of the biosynthetic molecules (44) in response to the subject interacting with the toilet paper.

3. The apparatus according to claim 1, wherein the substrate (26) comprises toilet paper, a tampon, a sanitary product, and/or a flushable pad, and wherein the substrate (26) is configured to receive vaginal fluids of the subject within reactive proximity of the biosynthetic molecules (44) in response to the subject interacting with substrate (26).

4. The apparatus according to any one of claims 1-3, wherein the biological sample comprises saliva, sweat, nasal mucus, mucus, phlegm, sweat, tears, volatile organic compounds that are present in the subject's body, epidermal tissue fluids, mucosal tissue fluids, and/or extracellular matrix (ECM).

5. The apparatus according to any one of claims 1-4, wherein the biosynthetic molecules (44) comprise one or more biosynthetic molecules (44) selected from the group consisting of: an aptamer, a molecularly imprinted polymer, an antibody, and a phage.

6. The apparatus according to any one of claims 1-5, wherein the substrate (26) further comprises an analog-to-digital converter (48) configured to convert the electrical energy generated by the converter (46) to a digital signal.

7. The apparatus according to any one of claims 1-6, wherein the apparatus is for use with a toilet bowl and a sensor module disposed within the toilet bowl, wherein the substrate (26) is configured to be placed in the toilet bowl, and wherein the wireless transmitter (48) is configured to transmit the signal by transmitting a signal that is detectable by the sensor module.

8. The apparatus according to any one of claims 1-7, wherein the biosynthetic molecules (44) are configured to undergo a biochemical interaction that generates thermal changes in the presence of the given biomarker, and wherein the converter (46) comprises a pyroelectric converter that is configured to convert the thermal changes generated by the biochemical interaction to electrical energy.

9. The apparatus according to any one of claims 1-7, wherein the biosynthetic molecules (44) are configured to undergo a biochemical interaction that generates spatial conformational changes in the presence of the given biomarker.

10. The apparatus according to claim 9, wherein the converter (46) comprises a piezoelectric converter that is configured to convert the spatial conformational changes generated by the biochemical interaction to electrical energy.

11. The apparatus according to any one of claims 1-7, wherein the biosynthetic molecules (44) are configured to undergo a biochemical interaction that generates electrochemical potential changes in the presence of the given biomarker.

12. The apparatus according to claim 11, wherein the converter (46) comprises one or more components selected from the group consisting of: a structural field-effect transistor and a potentiostat, wherein the one or more components are configured to convert the electrochemical potential changes generated by the biochemical interaction to electrical energy.

13. The apparatus according to any one of claims 1-12, further comprising:
a sensor configured to detect the signal generated by the substrate (26); and
a computer processor configured to generate an output in response to the detected signal.

14. The apparatus according to claim 14, wherein the substrate (26) is configured to transmit an electromagnetic signal that is indicative of a presence of troponin within urine of the subject, and wherein the computer processor is configured to generate an output indicating a likelihood of the subject suffering from cardiac ischemia in response thereto.

15. A method comprising:
placing a biological sample from a subject in reactive proximity with a plurality of biosynthetic molecules (44) disposed on a substrate (26), the biosynthetic molecules (44) configured to undergo a biochemical interaction in the presence of a given biomarker within the biological sample;
causing the biomarker, if present within the biological sample, to undergo the biochemical interaction with the biosynthetic molecules (44) such as to generate electrical energy; and
causing a transmitter (48) disposed upon the substrate (26) to generate a signal indicative of the generated electrical energy.
